# EUROPEAN PATENT SPECIFICATION

(11) **EP 3 391 381 B1**
(45) Date of publication and mention of the grant of the patent: **01.07.2020**
(21) Application number: 16719969.4
(22) Date of filing: 31.03.2016
(51) Int. Cl.: G21K 4/00, G01T 1/203

(54) **RADIATION SENSING THERMOPLASTIC COMPOSITE PANELS**
STRAHLUNGSEMPFINDLICHE THERMOPLASTISCHE VERBUNDPLATTEN
PANNEAUX COMPOSITES THERMOPLASTIQUES DE DÉTECTION DE RAYONNEMENT

(30) Priority: 14.12.2015 US 201562266860 P; 08.03.2016 US 201662304970 P
(43) Date of publication of application: 24.10.2018
(73) Proprietor: Carestream Dental Technology Topco Limited, London SW1Y 6RJ (GB)
(72) Inventor: JAGANNATHAN, Seshadri, Rochester, NY 14608 (US); RANKIN, Charles, M., Rochester, NY 14608 (US); FOLTS, Lawrence, D., Rochester, NY 14608 (US); ULREICH, Barbara, Rochester, NY 14608 (US); GUFFEY, Betsy, Rochester, NY 14608 (US); INGLESE, Jean-Marc, Rochester, NY 14608 (US)
(74) Representative: Wimmer, Hubert
(86) International application number: PCT/US2016/025120
(87) International publication number: WO 2017/105536

(56) References cited:
- EP-A1- 1 852 487
- US-A1- 2006 202 134
- US-A1- 2009 121 140

## Description

### FIELD OF THE INVENTION

The invention relates generally to the field of inorganic storage phosphor materials. More specifically, the invention relates to melt extrudable and/or injection moldable and/or hot-melt pressable composites of inorganic storage phosphor materials and thermoplastic and/or thermoset polymers and methods for making and/or using the same.

### BACKGROUND OF THE INVENTION

Near the beginning of the 20^{th} century, it was recognized that a medically useful anatomical image could be obtained when a film containing a radiation-sensitive silver halide emulsion is exposed to X-radiation (X-rays) passing through the patient. Subsequently, it was recognized that X-ray exposure could be decreased considerably by placing a radiographic phosphor panel adjacent to the film.

A radiographic phosphor panel typically contains a layer of an inorganic phosphor that can absorb X-rays and emit light to expose the film. The inorganic phosphor layer is generally a crystalline material that responds to X-rays in an image-wise fashion. Radiographic phosphor panels can be classified, based on the type of phosphors used, as prompt emission panels and image storage panels.

Image storage panels (also commonly referred to as "storage phosphor panels") typically contain a storage ("stimulable") phosphor capable of absorbing X-rays and storing its energy until subsequently stimulated to emit light in an image-wise fashion as a function of the stored X-ray pattern. A well-known use for storage phosphor panels is in computed or digital radiography. In these applications, the panel is first image-wise exposed to X-rays, which are absorbed by the inorganic phosphor particles, to create a latent image. While the phosphor particles may fluoresce to some degree, most of the absorbed X-rays are stored therein. At some interval after initial X-ray exposure, the storage phosphor panel is subjected to longer wave length radiation, such as visible or infrared light (e.g., stimulating light), resulting in the emission of the energy stored in the phosphor particles as stimulated luminescence (e.g., stimulated light) that is detected and converted into sequential electrical signals which are processed in order to render a visible image on recording materials, such as light-sensitive films or digital display devices (e.g., television or computer monitors). For example, a storage phosphor panel can be image-wise exposed to X-rays and subsequently stimulated by a laser having a red light or infrared beam, resulting in green or blue light emission that is detected and converted to electrical signals which are processed to render a visible image on a computer monitor. The stimulating light may also be other sources other than a laser (such as LED lamps), that would permit stimulation of a larger area of the storage phosphor, and the detection may be done using a two dimensional detector, such as a CCD or a CMOS device. Thereafter, images from storage phosphor panels can be "erased" by exposure to UV radiation, such as from fluorescent lamps.

Thus, storage phosphor panels are typically expected to store as much incident X-rays as possible while emitting stored energy in a negligible amount until after subsequent stimulation; only after being subjected to stimulating light should the stored energy be released. In this way, storage phosphor panels can be repeatedly used to store and transmit radiation images.

Attention is also drawn to US 2009 / 121 140 A1 disclosing a halide-containing stimulable phosphor precursor, a halide-containing stimulable phosphor, a radiation image conversion panel and a production method thereof.

Moreover, EP 1 852 487 A1 provides a preparation method of a precursor of a rare earth activated alkaline earth metal fluorohalide stimulable phosphor exhibiting superior plate characteristics, a rare earth activated alkaline earth metal fluorohalide stimulable phosphor obtained from the precursor and a radiation image conversion panel comprising the stimulable phosphor.

US 2006 / 202 134 A1 discloses a radiation image storage panel having a phosphor layer containing an energy-storing phosphor, in which the storage panel has a surface showing an absorbance of 0.2 to 0.5 at the stimulating wavelength of the phosphor, and the phosphor layer containing the phosphor at a packing density of 3.0 g/cm < 3 > or more.

Thus, there exists a need for improved storage phosphor panels. More specifically, there exists a need for melt extruded or injection molded or hot pressed inorganic storage phosphor panel has an image quality that is comparable to the image quality of the traditional solvent coated screen of equivalent x-ray absorbance.

### SUMMARY OF THE INVENTION

In accordance with the present invention, a free standing inorganic storage phosphor panel and a method of producing an inorganic storage phosphor panel as set forth in Claims 1 and 5, respectively, is provided. Further embodiments of the invention are inter alia disclosed in the dependent claims. An aspect of this application is to advance the art of medical, dental and non-destructive imaging systems.

Another aspect of this application is to address in whole or in part, at least the foregoing and other deficiencies in the related art.

It is another aspect of this application to provide in whole or in part, at least the advantages described herein.

Disclosed therein are melt extruded or injection molded or hot pressed inorganic storage phosphor panel embodiments including a melt extruded or injection molded or hot pressed inorganic storage phosphor layer comprising a thermoplastic polymer and an inorganic storage phosphor material, wherein the melt extruded or injection molded or hot pressed inorganic storage phosphor panel has an image quality that is comparable to or better than the image quality of the traditional solvent coated screen of equivalent x-ray absorbance.

Also disclosed therein are inorganic storage phosphor detection system embodiments including a melt extruded or injection molded or hot pressed inorganic storage phosphor panel comprising a melt extruded or injection molded or hot pressed inorganic storage phosphor layer comprising a thermoplastic olefin and an inorganic storage phosphor material.

Further disclosed therein are method embodiments of making a melt extruded or injection molded or hot pressed inorganic storage phosphor panel including providing thermoplastic polymer comprising at least one thermoplastic polymer and an inorganic storage phosphor material; and melt extruding or injection molding or hot pressing the thermoplastic polymer and the inorganic storage phosphor material to form a melt extruded or injection molded or hot pressed inorganic storage phosphor layer.

Further disclosed therein is an inorganic storage phosphor panel that can include an inorganic storage phosphor layer including at least one polymer, an inorganic storage phosphor material, where the inorganic storage phosphor material has 95% of the particles to be ≤ 6.8 microns in diameter and 95% of the particles to be ≥ 1.0 microns in diameter, where the storage phosphor layer has fewer than 5 defects per square cm.

Further disclosed therein is a method for an inorganic storage phosphor panel that can include melt extruding, injection molding or hot pressing materials comprising at least one polymer, and an inorganic storage phosphor material that has 95% of the particles to be ≤ 6.8 microns in diameter and 95% of the particles to be ≥ 1.0 microns in diameter, to form a manufactured inorganic storage phosphor layer, where the storage phosphor layer has fewer than 5 defects; exposing the manufactured inorganic storage phosphor layer to x-rays to form a latent image; and exposing the latent image in the manufactured inorganic storage phosphor layer to excitation light to generate a digital image of the latent image.

The invention is defined by the appended claims.

### BRIEF DESCRIPTION OF THE DRAWINGS

The foregoing and other objects, features, and advantages of the invention will be apparent from the following more particular description of the embodiments of the invention, as illustrated in the accompanying drawings. The elements of the drawings are not necessarily to scale relative to each other.

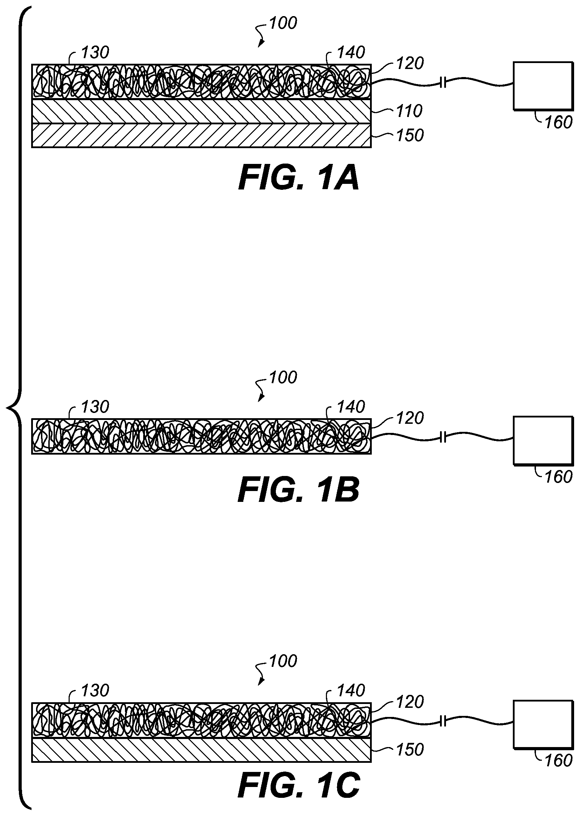

FIGS. 1A-1C depict exemplary portions of scintillator panels in accordance with various embodiments of the present disclosure.

### DESCRIPTION OF EXEMPLARY EMBODIMENTS

The following is a description of exemplary embodiments, reference being made to the drawings in which the same reference numerals identify the same elements of structure in each of the several figures.

Exemplary embodiments herein provide storage phosphor panels including an extruded storage phosphor layer with a thermoplastic polymer and a storage phosphor material, and methods of preparing thereof. It should be noted that while the present description and examples are primarily directed to radiographic medical imaging of a human or other subject, embodiments of apparatus and methods of the present application can also be applied to other radiographic imaging applications. This includes applications such as non-destructive testing (NDT), for which radiographic images may be obtained and provided with different processing treatments in order to accentuate different features of the imaged subject.

An important property of the screen is its x-ray absorbance. Depending on the specific application (orthopedic or mammography or intra- oral dental or extra oral dental or non-destructive testing of metals or...), the energy and the intensity of the radiation that is incident on the storage phosphor screen will be different. However, in order to be value as an x-ray imaging tool, the storage phosphor screen has to have sufficient x-ray absorbance, so as to produce a useful image. In practical terms, this requires that ∼ 40-60% of the extruded storage phosphor screen (by volume) be the storage phosphor material (barium fluorobromoiodide or cesium bromide).

Another requirement for the storage phosphor screen is that it be readable from either side of the screen, and in the transmission or the reflection mode, with respect to the direction of incidence of the stimulation radiation used for reading the information in the screen. And it would desirable that the screen can be handled under ambient lighting conditions or room light.

Depending on the specific imaging application (medical radiography or dental radiography or non-destructive testing), the physical characteristics required of the storage phosphor panel can be widely different. However, the divergent physical properties may be defined by a few key properties of the storage phosphor screen, such as its bending resistance (http://www.taberindustries.com/stiffness-tester), tear resistance (http://jlwinstruments.com/index.php/products/test-solutions/tear-resistance-testing/) or folding resistance (https://www.testingmachines.com/product/31-23-mit-folding-endurance-tester). A summary of various methods to measure these properties is outlined in (http://ipst.gatech.edu/faculty/popil_roman/pdf presentations/Prediction%20of%2 0Fold%20Cracking%20Propensity%20through%20Physical%20Testing.pdf). All this may be achieved using a single layer or a multi layered architecture, that would include additional, coextruded layers on the screen, that may contain particulates and/or chemistry to achieve the required physical properties needed to accommodate the mechanics of the scanner and/or handling by the end user. Further, it is important that the extruded storage phosphor screen be recyclable; i.e., it is necessary that the composition of the screen is such that they can re-used to make the storage phosphor screen, and/or the storage phosphor part of the screen can be reused to manufacture a new screen.

The stimulation wavelength and the emission wavelength of the storage phosphor panel are generally determined by the specific storage phosphor. The peak stimulation wavelength for the commonly used storage phosphors, the stimulation wavelength is fairly broad, and is in the region of 550-700 nm. However, the stimulated emission for the europium doped barium fluorobromoiodide storage phosphor has peak around 390 nm.

FIG. 1 depicts a portion of an exemplary storage phosphor panel 100 in accordance with various embodiments of the present disclosure. As used herein, "storage phosphor panel" is understood to have its ordinary meaning in the art unless otherwise specified, and refers to panels or screens that store the image upon exposure to X-radiation and emit light when stimulated by another (generally visible) radiation. As such, "panels" and "screens" are used interchangeably herein. It should be readily apparent to one of ordinary skill in the art that the storage phosphor panel 100 depicted in FIGS. 1A-1C represents a generalized schematic illustration and that other components can be added or existing components can be removed or modified.

Storage phosphor panels disclosed herein can take any convenient form provided they meet all of the usual requirements for use in computed radiography. As shown in FIG. 1A, the storage phosphor panel 100 may include a support 110 and a melt extruded or injection molded or hot pressed storage phosphor layer 120 disposed over the support 110. Any flexible or rigid material suitable for use in storage phosphor panels and does not interfere with the recyclability of storage phosphor screen can be used as the support 110, such as glass, plastic films, ceramics, polymeric materials, carbon substrates, and the like. In certain embodiments, the support 110 can be made of ceramic, (e.g., Al₂O₃,) or metallic (e.g., Al) or polymeric (e.g., polypropylene) materials. Also as shown in FIG. 1A, in an aspect, the support 110 can be coextruded with the storage phosphor layer 120. The support may be transparent, translucent, opaque, or colored (e.g., containing a blue or a black dye). Alternatively, if desired, a support can be omitted in the storage phosphor panel.

In another aspect, an anticurl layer may be coextruded on either side of the support, if a support is used, or on side of the storage phosphor screen, to manage the dimensional stability of the storage phosphor screen.

The thickness of the support 110 can vary depending on the materials used so long as it is capable of supporting itself and layers disposed thereupon. Generally, the support can have a thickness ranging from about 50 µm to about 1,000 µm, for example from about 80 µm to about 1000 µm, such as from about 80 µm to about 500 µm. The support 110 can have a smooth or rough surface, depending on the desired application. In an embodiment, the storage phosphor panel does not comprise a support.

The storage phosphor layer 120 can be disposed over the support 110, if a support is included. Alternatively, the storage phosphor layer 120 can be melt extruded or injection molded or hot pressed independently as shown in FIG. 1B, or melt extruded or injection molded or hot pressed together with an opaque layer, and anticurl layer, and combinations thereof, e.g., shown as layer 150, in FIG. 1A and FIG. 1C.

The storage phosphor layer 120 can include a thermoplastic polymer 130 and a storage phosphor material 140. The thermoplastic polymer 130 may be a polyolefin, such as polyethylene, a polypropylene, and combinations thereof, or a polyurethane, a polyester, a polycarbonate, a silicone, a siloxane, a polyvinyl chloride (PVC), a polyvinylidine chloride (PVdC). In an aspect, the polyethylene can be high density poly low density polyethylene (LDPE), medium density polyethylene (MDPE), linear low density polyethylene (LLDPE), very low density polyethylene (VLDPE), and the like. In a preferred embodiment, the thermoplastic polymer 130 is low density polyethylene (LDPE). The thermoplastic polymer 130 can be present in the storage phosphor layer 120 in an amount ranging from about 1% to about 50% by volume, for example from about 10% to about 30% by volume, relative to the total volume of the storage phosphor layer 120.

As used herein, "storage phosphor particles" and "stimulable phosphor particles" are used interchangeably and are understood to have the ordinary meaning as understood by those skilled in the art unless otherwise specified. "Storage phosphor particles" or "stimulable phosphor particles" refer to phosphor crystals capable of absorbing and storing X-rays and emitting electromagnetic radiation (e.g., light) of a second wavelength when exposed to or stimulated by radiation of still another wavelength. Generally, stimulable phosphor particles are turbid polycrystals having particle diameters of several micrometers to several hundreds of micrometers; however, fine phosphor particles of submicron to nano sizes have also been synthesized and can be useful. Thus, the optimum mean particle size for a given application is a reflection of the balance between imaging speed and desired image sharpness.

Stimulable phosphor particles can be obtained by doping, for example, rare earth ions as an activator into a parent material such as oxides, nitrides, oxynitrides, sulfides, oxysulfides, silicates, halides, and the like, and combinations thereof. As used herein, "rare earth" refers to chemical elements having an atomic number of 39 or 57 through 71 (also known as "lanthanoids"). Stimulable phosphor particles are capable of absorbing a wide range of electromagnetic radiation. In exemplary preferred embodiments, stimulable phosphor particles can absorb radiation having a wavelength of from about 0.01 to about 10 nm (e.g., X-rays) and from about 300 nm to about 1400 nm (e.g., UV, visible, and infrared light). When stimulated with stimulating light having a wavelength in the range of visible and infrared light, stimulable phosphor particles can emit stimulated light at a wavelength of from about 300 nm to about 650 nm.

Suitable exemplary stimulable phosphor particles for use herein include, but are not limited to, compounds having Formula (I):

MFX*_{1-z}*I*_{z}*uM^{a}X^{a}:yA:eQ:tD (I)

wherein M is selected from the group consisting of Mg, Ca, Sr, Ba, and combinations thereof;
X is selected from the group consisting Cl, Br, and combinations thereof;
M^{a} is selected from the group consisting of Na, K, Rb, Cs, and combinations thereof;
X^{a} is selected from the group consisting of F, Cl, Br, I, and combinations thereof;
A is selected from the group consisting of Eu, Ce, Sm, Th, Bi, and combinations thereof;
Q is selected from the group consisting of BeO, MgO, CaO, SrO, BaO, ZnO, Al₂O₃, La₂O₃, In₂O₃, SiO₂, TiO₂, ZrO₂, GeO₂, Nb₂O₅, Ta₂O₅, ThO₂, and combinations thereof;
D is selected from the group consisting of V, Cr, Mn, Fe, Co, Ni, and combinations thereof;
z is from about 0.0001 to about 1;
u is from about 0 to about 1;
y is from about 0.0001 to about 0.1;
e is from 0 to about 1; and
t is from 0 to about 0.01.

The amounts represented by "z", "u", "y", "e", and "t" are molar amounts. The same designations appearing elsewhere in this disclosure have the same meanings unless otherwise specified. In Formula (I), preferably, M is Ba; X is Br; M^{a} is selected from the group consisting of Na, K, and combinations thereof; X^{a} is selected from the group consisting of F, Br, and combinations thereof; A is Eu; Q is selected from the group consisting of SiO₂, Al₂O₃, and combinations thereof; and t is 0.

Other exemplary stimulable phosphor particles for use herein include, but are not limited to, compounds having Formula (II):

(Ba*_{1-a-b-c}*Mg*ₐ*Ca*_{b}*Sr*_{c}*)FX*_{1-z}*I*_{z}*rM^{a}X^{a}:yA:eQ:tD (II)

wherein X, M^{a}, X^{a}, A, Q, D e, t, z, and y are as defined above for Formula (I); the sum of a, b, and c, is from 0 to about 0.4; and r is from about 10⁻⁶ to about 0.1.

In Formula (II), preferably X is Br; M^{a} is selected from the group consisting of Na, K, and combinations thereof; X^{a} is selected from the group consisting of F, Br, and combinations thereof; A is selected from the group consisting of Eu, Ce, Bi, and combinations thereof; Q is selected from the group consisting of SiO₂, Al₂O₃, and combinations thereof; and t is 0.

Further exemplary stimulable phosphor particles for use herein include, but are not limited to, compounds having Formula (III):

m¹⁺X*ₐ*M²⁺X'₂*b*M³⁺X"3:cZ (III)

wherein M is selected from the group consisting of Li, na, K, Cs, Rb, and combinations thereof;
M²⁺ is selected from the group consisting of Be, Mg, Ca, Sr, Ba, Zn, Cd, Cu, Pb, Ni, and combinations thereof;
M³⁺ is selected from the group consisting of Sc, Y, La, Ce, Pr, Nd, Pm, Sm, Eu, Gd, Tb, Dy Ho, Er, Tm Yb, Lum Al, Bi, In, Ga, and combinations thereof;
Z is selected from the group consisting of Ga¹⁺, Ge²⁺, Sn²⁺, Sb³⁺, As³⁺, and combinations thereof;
X, X' and X" can be the same or different and each individually represents a halogen atom selected from the group consisting of F, Br, Cl, I; and 0 ≤ a ≤ 1; 0 ≤ b ≤ 1; 0 < c ≤ 0.2.

Preferred stimulable phosphor particles represented by Formulas (I), (II), or (III) include europium activated barium fluorobromides (*e.g*., BaFBr:Eu and BaFBrI:Eu), cerium activated alkaline earth metal halides, cerium activated oxyhalides, divalent europium activated alkaline earth metal fluorohalides, (e.g., Ba(Sr)FBr:Eu²⁺) divalent europium activated alkaline earth metal halides, rare earth element activated rare earth oxyhalides, bismuth activated alkaline metal halide phosphors, and combinations thereof.

An alternative to the Eu doped BaFBrI type storage phosphor is, Eu doped CsBr storage phosphor. This is generally used in the form a binderless storage phosphor screen, where the needle shaped Eu doped CsBr particles are generated by vapor deposition of the material on a substrate, which is then sealed water impermeable material. Such needle shaped europium doped cesium bromide storage phosphor screen has an emission peak around 450 nm.

The thermoplastic polymer and the inorganic storage phosphor material are melt compounded to form composite thermoplastic particles which are then melt extruded or injection molded or hot pressed to form the inorganic storage phosphor layer. For example, the composite thermoplastic particles can be prepared by melt compounding the thermoplastic polymer with the inorganic storage phosphor material using a twin screw compounder. The ratio of thermoplastic polymer to inorganic storage phosphor material (polymer: inorganic storage phosphor) can range from about 1:100 to about 1:0.01, by weight or volume, preferably from about 1:1 to about 1:0.1, by weight or volume. The composition comprises a copper phthalocyanine blue dye and may include inorganic, organic and/or polymeric additives to manage image quality and/or the physical properties of the extruded storage phosphor screen. Examples of the additives include, a blue dye (e.g., ultramarine blue, copper phthalocyanine,..) for managing image quality, surfactants (e.g., sodium dodecyl sulfate) for managing the colloidal stability of the storage phosphor particles, polymers (e.g., ethylene vinylacetate) for managing the rheology of the composite. During melt compounding, the thermoplastic polymer and the inorganic storage phosphor material can be compounded and heated through multiple heating zones. For example, in the case of polyolefins, the temperature of the heating zones can vary from ca. 170 °C - 250 °C, depending on the specific composition of the polymer/additive blends that are used, and the period of time in each zone depends on the polymer used and the temperature of the heating zone. Generally, the polymer can be heated for a time and temperature sufficient to melt the polymer and incorporate the inorganic storage phosphor material without decomposing the polymer. The period of time in each zone can range from about 1 second to about 1 minute. Upon exiting the melt compounder, the composite thermoplastic material can enter a water bath to cool and harden into continuous strands. The strands can be pelletized and dried at about 40°C. The screw speed and feed rates for each of the thermoplastic polymer 130 and the inorganic storage phosphor material 140 can be adjusted as desired to control the amount of each in the composite thermoplastic material.

The inorganic storage phosphor/thermoplastic polymer composite material is melt extruded or injection molded or hot pressed to form the inorganic storage phosphor layer in which the inorganic storage phosphor material is intercalated ("loaded") within the thermoplastic polymer. The inorganic storage phosphor/thermoplastic polymer composite layer is formed by melt extruding or injection molding or hot pressing the composite thermoplastic material. Without being limited by theory, it is believed that forming the inorganic storage phosphor/thermoplastic composite layer by melt extrusion or injection molding or hot pressing increases the homogeneity of the inorganic storage phosphor layer, and eliminates the undesirable "evaporated space" generated when the solvent is evaporated in the traditional solvent-coated panels. A melt extruded or injection molded or hot pressed inorganic storage phosphor/thermoplastic composite panel according to the present disclosure can have comparable image quality, as compared to the traditional solvent coated panels, along with improved mechanical and environmental robustness.

In the case of the inorganic storage phosphor/thermoplastic polymer composite layer being melt extruded or injection molded or hot pressed in combination with a support layer, the melt processing parameters (temperature, screw speed and pump speed in the case of melt extrusion and injection molding, and temperature and pressure in the case of hot pressing) can be adjusted to control the thickness for each of the inorganic storage phosphor/thermoplastic polymer composite layer and the support layer, individually.

The thickness of the inorganic storage phosphor /thermoplastic composite layer can range from about 10 µm to about 1000 µm, preferably from about 50 µm to about 750 µm, more preferably from about 100 µm to about 500 µm.

Optionally, the melt extruded or injection molded or hot pressed inorganic storage phosphor panel can include a protective overcoat disposed over the inorganic storage phosphor/thermoplastic composite layer, which provides enhanced mechanical strength and scratch and moisture resistance, if desired.

In an embodiment, a scintillation detection system can include the disclosed storage phosphor panel 100 coupled, inserted or mounted to at least one storage phosphor panel reader/scanner 160. Choice of a particular storage phosphor reader will depend, in part, on the type of storage phosphor panel being fabricated and the intended use of the ultimate device used with the disclosed storage phosphor panel.

### IMAGE QUALITY ASSESSMENT

The image quality assessments were done as described below. The extruded plate is adhered to a black PET (Toray Lumirror X30 - 10mil) support using an optically clear adhesive (3M 8141). This plate is then placed in the Carestream CS2200 x-ray generator, and exposed to an x-ray exposure of 70kV, 7 mA, 0.16 sec. This exposed plate, kept in subdued ambient light, is then scanned in the Carestream Health CS7600 intra oral dental scanner, in the super high resolution mode. The image is saved as a JPEG file and looked at on a computer monitor for defect count. The plates are all dental size 2 with an area of approximately 12.71 cm².

Applicants have found that inorganic storage phosphor particles disclosed herein suffer nano-particle effects when reaching sizes below 1 micron including increased viscosity, increased surface roughness including holes and recesses on surfaces and/or agglomeration effects.

### COMPARATIVE EXAMPLE 1

### COMPOSITE THERMOPLASTIC PARTICLE PRODUCTION

Inorganic storage phosphor/thermoplastic composite pellets according to the present disclosure were prepared comprising of 86 % wt.barium flurobromoiodide (BFBrI) and 14% wt low density polyethylene (LDPE EM811A, available from Westlake Longview Corp. of Houston, TX).
The inorganic storage phosphor particles was characterized using the Microtrac 9200 FRA, to have 95% of the particles to be ≤ 8.33 microns and 50% of the particles to be ≤ 4.12 microns in diameter.
The formulation included a blue dye (copper phthalocyanine) at a level of 100 ppm with respect to the weight of the phosphor. A 10% concentration of 65530-A Trans Blue (copper phthalocyanine) in LDPE EM 811AA was diluted step wise to a concentration of 1% with the LDPE EM811A, available from Westlake Longview Corp. of Houston, TX. To achieve the 100 ppm blue dye concentration in the final inorganic storage phosphor/thermoplastic polymer composite, the undyed EM811A polymer resin and the dyed (1% blue) EM811A masterbatch were blended and compounded with the the BFBrI powder using a Leistritz twin screw compounder.
The die temperature was set to 220°C and 10 heating zones within the compounder were set to the temperatures shown in Table 1 below:

**TABLE 1**

| **Zone** | 1 | 2 | 3 | 4 | 5 | 6 | 7 | 8 | 9 | 10 |
|---|---|---|---|---|---|---|---|---|---|---|
| **Temp (°C)** | **220** | **220** | **220** | **220** | **220** | **220** | **220** | **220** | **220** | **220** |

After exiting the die, the inorganic storage phosphor/thermoplastic composite pellets, comprising LDPE loaded with BFBrI, entered a 25°C water bath to cool and hardened into continuous strands. The strands were then fed into a pelletizer and dried at 40 °C.

### EXTRUSION OF INORGANIC STORAGE PHOSPHOR LAYER

The pelletized composite thermoplastic materials were loaded into a single screw Davis Standard extruder. Within the extruder, heating zones were set to the temperatures shown in Table 2

**TABLE 2**

| **Davis Extruder** | |
|---|---|
| **Zone** | **Temp** |
| 1 | 390°F |
| 2 | 400°F |
| 3 | 430°F |
| 4 | 430°F |
| Gate | 430°F |
| Adapter | 430°F |
| Poly line | 430°F |
| Melt pump | 430°F |

The pelletized material (composite thermoplastic) was extruded through a single die with the die temperature set at 430°F form an extruded inorganic storage phosphor panel in the thickness range of 100-200 microns.

### COMPARATIVE EXAMPLE 2

### COMPOSITE THERMOPLASTIC PARTICLE PRODUCTION

Inorganic storage phosphor/thermoplastic composite pellets according to the present disclosure were prepared comprising 83 % wt.barium flurobromoiodide (BFBrI) and 17% wt low density polyethylene (LDPE EM811A, available from Westlake Longview Corp. of Houston, TX).

The following differences between comparative example 1 and example 2 is the weight of the phosphor is 86% versus 83%, there is no blue dye, and the inorganic storage phosphor particles as characterized using the Microtrac 9200 FRA, to have 95% of the particles to be ≤ 7.56 microns and 50% of the particles to be ≤ 4.20 microns in diameter.

The die temperature was set to 220°C and 10 heating zones within the compounder were set to the temperatures shown in Table 3 below:

**TABLE 3**

| | | | | | | | | | | |
|---|---|---|---|---|---|---|---|---|---|---|
| **Zone** | 1 | 2 | 3 | 4 | 5 | 6 | 7 | 8 | 9 | 10 |
| **Temp (°C)** | **220** | **220** | **220** | **220** | **220** | **220** | **220** | **220** | **220** | **220** |

After exiting the die, the inorganic storage phosphor/thermoplastic composite pellets, comprising LDPE loaded with BFBrI, entered a 25°C water bath to cool and hardened into continuous strands. The strands were then fed into a pelletizer and dried at 40 °C.

### EXTRUSION OF INORGANIC STORAGE PHOSPHOR LAYER

The pelletized composite thermoplastic materials were loaded into a single screw Davis Standard extruder. Within the extruder, heating zones were set to the temperatures shown in Table 4

**TABLE 4**

| **Davis Standard Extruder** | |
|---|---|
| **Zone** | **Temp** |
| 1 | 390°F |
| 2 | 400°F |
| 3 | 430°F |
| 4 | 430°F |
| Gate | 430°F |
| Adapter | 430°F |
| Poly line | 430°F |
| Melt pump | 430°F |

The pelletized material (composite thermoplastic) was extruded through a single die with the die temperature set at 430°F form an extruded inorganic storage phosphor panel in the thickness range of 100-200 microns.

### REFERENCE EXAMPLE 1

### COMPOSITE THERMOPLASTIC PARTICLE PRODUCTION

A sample was prepared as described in comparative example 1, with the following differences between comparative example 1 and inventive example 1 is there is no blue dye, and the inorganic storage phosphor particles as characterized using the Microtrac 9200 FRA, to have 95% of the particles to be ≤ 6.78 microns and 50% of the particles to be ≤ 3.81 microns in diameter.
The die temperature was set to 220°C and 10 heating zones within the compounder were set to the temperatures shown in Table 5 below:

**TABLE 5**

| **Zone** | 1 | 2 | 3 | 4 | 5 | 6 | 7 | 8 | 9 | 10 |
|---|---|---|---|---|---|---|---|---|---|---|
| **Temp (°C)** | **220** | **220** | **220** | **220** | **220** | **220** | **220** | **220** | **220** | **220** |

After exiting the die, the composite thermoplastic particles, comprising of LDPE loaded with BFBrI, entered a 25°C water bath to cool and hardened into continuous strands. The strands were then pelletized in a pelletizer and dried at 40°C.

### EXTRUSION OF INORGANIC STORAGE PHOSPHOR LAYER

The pelletized composite thermoplastic materials were loaded into a single screw Davis Standard extruder. Within the extruder, heating zones were set to the temperatures shown in Table 6

**TABLE 6**

| **Davis Standard Extruder** | |
|---|---|
| **Zone** | **Temp** |
| 1 | 390°F |
| 2 | 400°F |
| 3 | 430°F |
| 4 | 430°F |
| Gate | 430°F |
| Adapter | 430°F |
| Poly line | 430°F |
| Melt pump | 430°F |

The pelletized material (composite thermoplastic) was extruded through a single die with the die temperature set at 430°F form an extruded inorganic storage phosphor panel in the thickness range of 100-200 microns.

### INVENTIVE EXAMPLE 2

### COMPOSITE THERMOPLASTIC PARTICLE PRODUCTION

A sample was prepared as described in comparative example 1, with the primary differences being: the blue dye (copper phthalocyanine) level is approximately 200 ppm with respect to the weight of the phosphor and the inorganic storage phosphor particles was characterized using the Microtrac 9200 FRA, to have 95% of the particles to be ≤ 6.00 microns and 50% of the particles to be ≤ 3.45 microns in diameter.

### EXTRUSION OF INORGANIC STORAGE PHOSPHOR LAYER

The pelletized composite thermoplastic materials were loaded into a single screw Davis Standard extruder. Within the extruder, heating zones were set to the temperatures shown in Table 7

**TABLE 7**

| **Davis Standard Extruder** | |
|---|---|
| **Zone** | **Temp** |
| 1 | 390°F |
| 2 | 400°F |
| 3 | 430°F |
| 4 | 430°F |
| Gate | 430°F |
| Adapter | 430°F |
| Poly line | 430°F |
| Melt pump | 430°F |

The pelletized material (composite thermoplastic) was extruded through a single die with the die temperature set at 430°F form an extruded inorganic storage phosphor panel in the thickness range of 100-200 microns.

**The comparative and inventive examples were characterized for defects as described above.**

| | **# defects per cm²** |
|---|---|
| **Comparative example 1** | ≥30 |
| **Comparative example 2** | ≥30 |
| Reference **example 1** | ≤ 5 |
| **Inventive example 2** | ≤ 5 |

The term "at least one of' is used to mean one or more of the listed items can be selected. The term "about" indicates that the value listed can be somewhat altered, as long as the alteration does not result in nonconformance of the process or structure to the illustrated embodiment.

## Claims

1. A free standing inorganic storage phosphor panel (100) comprising:
an inorganic storage phosphor layer (120) comprising at least one polymer (130), an inorganic storage phosphor material (140) and a copper phthalocyanine blue dye;
wherein the inorganic storage phosphor layer (120) is formed by melt compounding the at least one polymer (130), inorganic storage phosphor material (140), and copper phthalocyanine blue dye to form a melt and by then melt extruding, injection molding, or hot pressing the melt compounded material;
wherein the inorganic storage phosphor material (140) comprises particles in which 95% of the particles are less than or equal to 6.8 microns in diameter and 95% of the particles are greater than or equal to 1.0 microns in diameter; and
wherein the storage phosphor layer (120) has fewer than 5 defects per square cm.

2. The storage phosphor panel (100) of claim 1 further comprising a support (110) comprising at least one polymer, the support being transparent, translucent, opaque or colored.

3. The storage phosphor panel (100) of claim 1, wherein 50% of the inorganic storage phosphor material particles are less than 3.8 microns in diameter.

4. The storage phosphor panel (100) of claim 1, wherein the polymer (130) comprises at least one thermoplastic polyolefin.

5. A method of producing an inorganic storage phosphor panel (100) comprising:
fabricating an inorganic storage phosphor layer (120) by melt compounding an inorganic storage phosphor material including at least one polymer (130), an inorganic storage phosphor material (140) and a copper phthalocyanine blue dye to produce a melt;
melt extruding, injection molding, or hot pressing the melt compounded material to form a manufactured inorganic storage phosphor panel (100);
wherein the inorganic storage phosphor material (140) comprises particles in which 95% of the particles are less than or equal to 6.8 microns in diameter and 95% of the particles are greater than or equal to 1.0 microns in diameter, wherein the storage phosphor layer (120) has fewer than 5 defects per square cm.

6. The method of claim 5, wherein 50% of the inorganic storage phosphor material (140) particles are less than 3.8 microns in diameter.

7. The method of claim 5, where the polymer comprises at least one thermoplastic polyolefin.

## Patentansprüche

1. Eine freistehende, anorganische Speicher-Phosphor- bzw. Leuchtstoffplatte (100), die Folgendes aufweist:
eine anorganische Speicher-Phosphor- bzw. Leuchtstoffschicht (120), die mindestens ein Polymer (130), ein anorganisches Speicher-Phosphor- bzw. Leuchtstoffmaterial (140) und einen blauen Kupferphthalocyanin-Farbstoff aufweist;
wobei die anorganische Speicher-Leuchtstoffschicht (120) gebildet wird durch Schmelzcompoundieren des mindestens einen Polymers (130), des anorganischen Speicher-Leuchtstoffmaterials (140) und des blauen Kupferphthalocyanin-Farbstoffs, um eine Schmelze zu bilden, und durch anschließendes Schmelzextrudieren, Spritzgießen oder Heißpressen des schmelzcompoundierten Materials;
wobei das anorganische Speicher-Leuchtstoffmaterial (140) Partikel aufweist, bei denen 95 % der Partikel einen Durchmesser von weniger als oder gleich 6,8 Mikrometern aufweisen und bei denen 95 % der Partikel einen Durchmesser von mehr als oder gleich 1,0 Mikrometern aufweisen; und
wobei die Speicher-Leuchtstoffschicht (120) weniger als 5 Defekte pro Quadratzentimeter aufweist.

2. Die Speicher-Leuchtstoffplatte (100) nach Anspruch 1, die ferner einen Träger (110) aufweist, der mindestens ein Polymer aufweist, wobei der Träger transparent, transluzent, opak oder gefärbt ist.

3. Die Speicher-Leuchtstoffplatte (100) nach Anspruch 1, wobei 50 % der anorganischen Speicher-Leuchtstoffmaterialpartikel einen Durchmesser von weniger als 3,8 Mikrometern aufweisen.

4. Die Speicher-Leuchtstoffplatte (100) nach Anspruch 1, wobei das Polymer (130) mindestens ein thermoplastisches Polyolefin aufweist.

5. Ein Verfahren zur Herstellung einer anorganischen Speicher-Phosphor- bzw. Leuchtstoffplatte (100), das Folgendes aufweist:
Erzeugen einer anorganischen Speicher-Phosphor- bzw. Leuchtstoffschicht (120) durch Schmelzcompoundieren eines anorganischen Speicher-Phosphor- bzw. Leuchtstoffmaterials, das mindestens ein Polymer (130), ein anorganisches Speicher-Phosphor- bzw. Leuchtstoffmaterial (140) und einen blauen Kupferphthalocyanin-Farbstoff enthält, um eine Schmelze herzustellen;
Schmelzextrudieren, Spritzgießen oder Heißpressen des schmelzcompoundierten Materials, um eine gefertigte anorganische Speicher-Leuchtstoffplatte (100) zu bilden;
wobei das anorganische Speicherleuchtstoffmaterial (140) Partikel aufweist, bei denen 95 % der Partikel einen Durchmesser von weniger als oder gleich 6,8 Mikrometern aufweisen und bei denen 95 % der Partikel einen Durchmesser von mehr als oder gleich 1,0 Mikrometern aufweisen, wobei die Speicher-Leuchtstoffschicht (120) weniger als 5 Defekte pro Quadratzentimeter aufweist.

6. Das Verfahren nach Anspruch 5, wobei 50 % der Partikel des anorganischen Speicher-Leuchtstoffmaterials (140) einen Durchmesser von weniger als 3,8 Mikrometern aufweisen.

7. Das Verfahren nach Anspruch 5, wobei das Polymer mindestens ein thermoplastisches Polyolefin aufweist.

## Revendications

1. Panneau fluorescent de stockage inorganique autonome (100) comprenant :
une couche fluorescente de stockage inorganique (120) comprenant au moins un polymère (130), un matériau fluorescent de stockage inorganique (140) et un colorant bleu de phtalocyanine de cuivre ;
dans lequel la couche fluorescente de stockage inorganique (120) est formée par la composition par fusion dudit au moins un polymère (130), du matériau fluorescent de stockage inorganique (140), et du colorant bleu de phtalocyanine de cuivre pour former un fondu puis par extrusion par fusion, moulage par injection, ou pression à chaud du matériau composé par fusion ;
dans lequel le matériau fluorescent de stockage inorganique (140) comprend des particules parmi lesquelles 95 % des particules ont un diamètre inférieur ou égal à 6,8 microns et 95 % des particules ont un diamètre égal ou supérieur à 1,0 microns ; et
dans lequel la couche fluorescente de stockage (120) a moins de 5 défauts par cm carré.

2. Panneau fluorescent de stockage (100) selon la revendication 1, comprenant en outre un support (110) comprenant au moins un polymère, le support étant transparent, translucide, opaque ou coloré.

3. Panneau fluorescent de stockage (100) selon la revendication 1, dans lequel 50 % des particules du matériau fluorescent de stockage inorganique ont un diamètre inférieur à 3,8 microns.

4. Panneau fluorescent de stockage (100) selon la revendication 1, dans lequel le polymère (130) comprend au moins une polyoléfine thermoplastique.

5. Procédé de production d'un panneau fluorescent de stockage inorganique (100) comprenant les étapes suivantes :
la fabrication d'une couche fluorescente de stockage inorganique (120) par la composition par fusion d'un matériau fluorescent de stockage inorganique comportant au moins un polymère (130), un matériau fluorescent de stockage inorganique (140) et un colorant bleu de phtalocyanine de cuivre pour produire un fondu ;
l'extrusion par fusion, le moulage par injection, ou la pression à chaud du matériau composé par fusion pour former un panneau fluorescent de stockage inorganique fabriqué (100) ;
dans lequel le matériau fluorescent de stockage inorganique (140) comprend des particules parmi lesquelles 95 % des particules ont un diamètre inférieur ou égal à 6,8 microns et 95 % des particules ont un diamètre égal ou supérieur à 1,0 microns, dans lequel la couche fluorescente de stockage (120) a moins de 5 défauts par cm carré.

6. Procédé selon la revendication 5, dans lequel 50 % des particules du matériau fluorescent de stockage inorganique (140) ont un diamètre inférieur à 3,8 microns.

7. Procédé selon la revendication 5, dans lequel le polymère comprend au moins une polyoléfine thermoplastique.
